# EUROPEAN PATENT SPECIFICATION

(11) **EP 2 934 682 B1**
(45) Date of publication and mention of the grant of the patent: **17.10.2018**
(21) Application number: 13815827.4
(22) Date of filing: 24.12.2013
(51) Int. Cl.: A61Q 5/06, A61Q 5/12, A61K 8/73

(54) **PERSONAL CARE COMPOSITION COMPRISING A NATURAL FILM-FORMING BIOPOLYMER AND METHODS OF MAKING THE SAME**
KÖRPERPFLEGEZUSAMMENSETZUNG ENTHALTEND EIN NATÜRLICHES FILM-FORMENDES BIOPOLYMER AND METHODEN DEREN HERSTELLUNG
COMPOSITION D'HYGIÈNE PERSONNELLE COMPRENANT UN POLYMÈRE FILMOGÈNE NATUREL ET SES PROCÉDÉS DE FABRICATION

(30) Priority: 24.12.2012 GB 201223374
(43) Date of publication of application: 28.10.2015
(73) Proprietor: Keracol Limited, Leeds LS2 9JT (GB)
(72) Inventor: BLACKBURN, Richard Simon, Leeds Yorkshire LS2 9JT (GB); RAYNER, Christopher Mark, Leeds Yorkshire LS2 9JT (GB)
(74) Representative: HGF Limited
(86) International application number: PCT/GB2013/053430
(87) International publication number: WO 2014/102545

(56) References cited:
- WO-A1-2005/110341
- FR-A1- 2 925 310
- JP-A- S62 114 906
- US-A- 3 988 438
- DATABASE GNPD [Online] MINTEL; December 2006 (2006-12), Shiseido: "Mothers Milk Conditioner (Shiseido Super Mild)", XP002726287, Database accession no. 626397
- DATABASE GNPD [Online] MINTEL; July 2012 (2012-07), Hain Celestial Group: "Curl Shaping Creme: Queen Helene Royal Curl", XP002726288, Database accession no. 1836427

## Description

This invention relates to a personal care composition comprising a natural film-forming biopolymer. Particularly, the invention relates to a personal care composition for application to a keratinous substrate such as human hair. Methods of making and applying the composition are also disclosed.

### BACKGROUND

Personal care compositions incorporate a variety of polymers to provide aesthetic and feel benefits to consumers. Typically, personal care compositions such as hair styling products are based on synthetic polymers and synthetic copolymers including, but not limited to, poly(vinylpyrrolidone), polyquaternium-11, and polyquaternium-16. Polyquaternium-10 is also extensively used, which is based on plant-derived starch, but is subsequently significantly modified through synthetic chemical processes. The above-noted polymers therefore usually contain at least one key component part of their structure that is ultimately derived from petrochemical precursors. These polymers and copolymers are generally water and/or ethanol soluble and provide properties such as hold and film formation. In recent years, development of new synthetic polymers has enhanced overall product performance and benefit efficacy.

In view of the rapid worldwide depletion of petrochemical feedstocks, attention has increasingly turned to the use of natural biopolymers or naturally derived polymers that would offer a more sustainable future. Natural biopolymers are polymers that are derived directly from plant or animal matter, insofar as the biopolymer is extracted from plant or animal matter in the general form in which it occurs *in planta* or *in vivo* (*e.g.* cellulose, starch, alginic acid, pectin, chitin). Naturally derived polymers are polymers that are derived indirectly from plant or animal matter, insofar the polymer is synthesised or derived from chemicals extracted or produced from plant or animal matter (*e*.*g*. polylactic acid). Despite developments in the synthetic polymer space, research in the natural polymer space has been comparatively limited.

In addition to polyquaternium-10, current natural and naturally-derived alternatives to these products are also largely based on corn starch and modified corn starch. However, the existing natural and naturally-derived polymers exhibit major disadvantages including lack of ethanol solubility, opaque film formation and leave a starchy feel on the hair. Furthermore, they are generally expensive and therefore limit commercial formulation possibilities. Polyquaternium-10 is used extensively in the cosmetics industry in hair styling products and is prepared by derivatisation of starch with *N*-(2,3-epoxypropyl)-*N*,*N*,*N-*trimethylammonium chloride (glytac A) to make hydroxypropyltriammonium starch. Although polyquaternium-10 is synthesised from a naturally occurring source, the use of glytac A in its preparation presents problems from an environmental and toxicological perspective as there is evidence which demonstrates glytac A is an Ames test positive carcinogen.

In addition to polyquaternium-10, the prior art discloses a number of personal care compositions incorporating naturally-derived polymers and processes to prepare the same. EP948960 describes a process for derivatisation of corn starch to prepare a non-ionically modified starch product wherein the starch may be additionally hydrolyzed. In the process corn starch is treated with alkali and then modified with up to 15% propylene oxide. It is reported that the use of such starches provides a clear solution with a low viscosity, and good pump spray characteristics. Further, the disclosure in EP948960 asserts that the resultant composition provides a clear film which is not tacky, has good stiffness and improved humidity resistance.

AkzoNobel/National Starch market a polymer called *AMAZE*®*.* In terms of hair styling performance, when used on its own *AMAZE*® exhibits good initial strength and excellent film flexibility, which makes it a reliable choice for creating styling formulations that provide a more natural, flexible, long-lasting hold.

Cationic polyelectrolytes are used as hair-fixative polymers and preferred gellants are anionic polyelectrolytes. However, successfully combining the properties of cationic and anionic polyelectrolytes is difficult to achieve because mixing solutions of cationic and anionic polyelectrolytes usually causes the two polymers to interact and separate from solution as a complex.

US20060183822 describes the use of a polyampholyte as one of the components, which is a polymer that contains both cationic and anionic groups on the same molecule. In this context, styling and/or fixing can be obtained simultaneously with good hair disentangling and feel by combining hair fixative polymers with an amphoteric starch; e.g. starch modified by (2-chloroethyl) aminodipropionic acid as described in US4017460, or other modifications as described in US5455340.

Other natural biopolymers include chitin and chitosan which are abundantly available in nature, but tend to be limited with regard to their solubility properties. Chitin in particular is difficult to formulate in personal care compositions without significant derivatisation. Such compounds therefore typically require further significant chemical modification to enhance their utility.

DE19619111 describes the use of a modified chitin in hair styling products. Chitin 6-O-substituted with C₂₋₂₀ straight- or branched-chain α-hydroxyalkyl groups is a film-forming polymer useful in cosmetic compositions for setting the hair. These chitin derivatives are water soluble, do not form excessive residues on the hair, are compatible with cationic and anionic polymers, are biodegradable, and impart a natural feel to the hair. They can also be used as thickening and gel-forming agents. Thus, refluxing chitin with NaOH and propylene oxide produces hydroxypropylchitin which can be delivered from an aerosol can with butane (92:8) for use as a hair-setting foam.

DE19857546, DE19531145, DE19524125, DE92-4234743, WO2009006210, WO2009006212 and WO0025734 all disclose the use of chitosan in hair styling preparations. The greater water solubility (at acidic pH) of chitosan in comparison with chitin offers greater possibilities for the use of the unmodified polymer in cosmetic formulations. Water-soluble chitosan salts can be incorporated into compositions having excellent properties for fixing hair, and form hard, non-adhesive films which can exchange water vapour with the atmosphere surrounding them without altering their properties. It has been reported that the concentration of chitosan salts necessary for fixing hair is comparatively lower (ca. 1% weight of formulation) than that of conventional resins (ca. 3%).

US3988438 describes shampoo compositions that are a combination of natural soaps, a syndet and an alginate salt.

FR2925310 described a kit, containing two compositions, for coating keratin fibers. One of the compositions comprises an aqueous phase and an emulsifier system comprising at least 1% of triethanolamine and at least one compound based on alginic acid.

The company Shiseido have marketed 'Mother's Milk Conditioner' which contains a variety of ingredients, including alginic acid.

The company Hain Celestial Group have marketed 'Queen Helene Royal Curl' which contains a variety of ingredients, including pectin.

JP62114906 describes a cosmetic comprising a quaternary ammonium salt of alginic acid.

Despite the existence of personal care compositions comprising natural biopolymers or naturally derived polymers there are needed additional alternatives in order to satisfy the demand for sustainable ingredients in such products. With this objective in mind, there is an increasing desire for personal care compositions with principal ingredients that are not ultimately derived from petrochemical precursors. Furthermore, there is a need for a personal care composition which successfully incorporates a natural biopolymer, without covalent chemical modification, whilst still exhibiting the desired properties of applicability, hold, adhesion, transparency and washability following application to a keratinous substrate.

### BRIEF SUMMARY OF THE DISCLOSURE

The present invention relates to a personal care composition comprising a natural film-forming biopolymer and a water-containing solvent wherein said composition further comprises a compound containing an amine moiety and wherein said solvent is an alcohol/water mixture wherein said mixture comprises at least 15% alcohol by weight of the composition; wherein said alcohol has a boiling point of less than 100 °C; and wherein said biopolymer is alginic acid or pectin. A natural biopolymer in the context of the invention is one which is derived directly from plant or animal matter.

Thus in a first aspect of the invention, there is provided a personal care composition comprising a natural film-forming biopolymer and a water-containing solvent wherein said composition can further comprise a compound containing an amine moiety. Advantageously, the composition of the invention includes a compound containing an amine moiety in addition to the biopolymer in order to enable the formation of films with high clarity. This is in contrast to personal care compositions of the prior art that incorporate starch-based polymers whereby opaque films are formed which are considered undesirable to consumers. Furthermore, the presence of the compound containing an amine moiety improves the hydration of the biopolymer.

In an embodiment, said biopolymer reacts with said compound containing an amine moiety to form a salt.

In an embodiment, said compound containing an amine moiety is an alkylamine, a hydroxyalkylamine or an amino acid. In some embodiments said compound containing an amine moiety is an alkylamine, a hydroxyalkylamine or a proteinogenic amino acid or derivative thereof. A proteinogenic amino acid can be defined as an amino acid which is a precursor to a protein. This is in contrast to a non-proteinogenic amino acid which is not incorporated in proteins.

In an embodiment, said compound containing an amine moiety is selected from the group consisting of: an amine, diamine, triamine, polyamine, aminoalcohol, diaminoalcohol, triaminoalcohol and polyaminoalcohol.

In an embodiment, said compound containing an amine moiety is selected from the group consisting of: a primary amine, secondary amine, tertiary amine, primary aminoalcohol, secondary aminoalcohol and tertiary aminoalcohol, wherein said primary aminoalcohol, secondary aminoalcohol, tertiary aminoalcohol have the general structure according to Formula (I), Formula (II) or Formula (III): 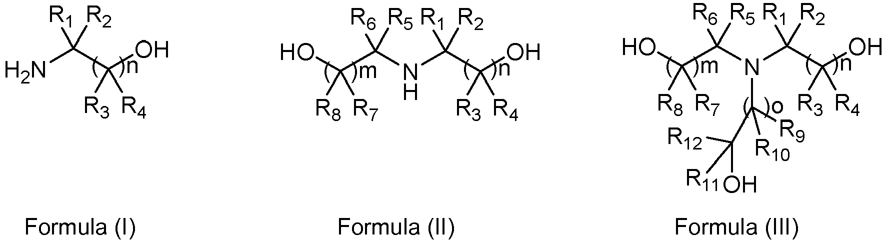 wherein R₁ to R₁₂ are each independently selected from the group consisting of: H, C₁₋₁₂ alkyl, aryl, C₁₋₁₂ alkyl aryl, and C₁₋₁₂ heteroalkyl wherein said C₁₋₁₂ heteroalkyl contains one of more heteroatoms selected from oxygen, nitrogen or sulphur; and m, n and o are each independently an integer from 0 to 14.

In an embodiment, said compound containing an amine moiety is selected from the group consisting of: ethanolamine, ethanolamine derivatives, triethanolamine, triethanolamine derivatives, aminomethylpropanol, aminomethylpropanol derivatives, isopropanolamine, isopropanolamine derivatives, ethylene diamine, ethylene diamine derivatives, propylene diamine, propylene diamine derivatives, ethylenediaminetetraacetic acid and salts of ethylenediaminetetraacetic acid.

In an embodiment, said compound containing an amine moiety is triethanolamine or 2-amino-2-methyl-1-propanol.

In another embodiment, said compound containing an amine moiety is arginine, histidine, proline, glycine or lysine and derivatives thereof wherein said derivatives of arginine, histidine, proline, glycine or lysine include salts, zwitterions, polymers, esters or amides.

In some embodiments, said compound containing an amine moiety is an amino acid and derivatives thereof, wherein the amino acid has the general structure according to Formula (IV): 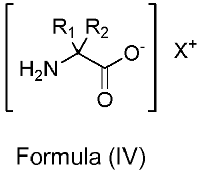 wherein R₁ and R₂ are each independently selected from the group consisting of: H, C₁₋₁₂ alkyl, aryl (e.g. phenyl), C₁₋₁₂ alkyl aryl and C₁₋₁₂ heteroalkyl wherein said C₁₋₁₂ heteroalkyl contains one or more heteroatoms selected from oxygen, nitrogen or sulphur and wherein X⁺ is a counter ion. In an embodiment X⁺ can be hydrogen.

In an embodiment, the composition of the invention comprises said biopolymer in an amount of about 0.1% to about 15% by weight of the composition. In another embodiment the composition of the invention comprises said biopolymer in an amount of about 0.1% to about 10% by weight of the composition and in other embodiments in an amount of about 0.1% to about 5% by weight of the composition.

In an embodiment, the composition of the invention comprises said compound containing an amine moiety in an amount of about 0.1% to about 3% by weight of the composition.

The solvent of the composition is an alcohol/water mixture that comprises at least 15% alcohol by weight of the composition. In embodiments, said mixture comprises at least 20% alcohol by weight of the composition. In further embodiments, said mixture may respectively comprise at least 30%, at least 40% or at least 50% alcohol by weight of the composition.

In an embodiment, said mixture comprises 30% to 60% alcohol by weight of the composition. In other embodiments, said mixture comprises 40% to 60% alcohol by weight of the composition. In other embodiments, said mixture comprises about 45% to about 55% by weight of the composition and in further embodiments said mixture comprises about 50% to about 55% by weight of the composition. In certain embodiments, compositions of the invention advantageously contain higher proportions of ethanol as part of the solvent system than natural biopolymer-based personal care compositions of the prior art.

The alcohol has a boiling point of less than 100 °C. In certain embodiments, the alcohol is ethanol.

In an embodiment, said composition comprises at least 20% water by weight of the composition. In other embodiments, said composition comprises at least 30% water by weight of the composition. In some embodiments said composition comprises at least 40% water by weight of the composition.

In other embodiments, said composition comprises at least 90% water by weight of the composition.

In an embodiment, said composition is substantially free from synthetic polymers or petrochemically derived polymers. In this context, the term "synthetic polymers" refers to any polymer that cannot be found in nature and can only be obtained via chemical synthesis. Petrochemically derived polymers contain at least one key component part of their structure that is ultimately derived from petrochemical precursors.

In an embodiment, said composition may further comprise one or more ingredients selected from the group consisting of: preservatives (e.g. sodium benzoate, phenoxyethanol, methylisothiazolinone), conditioning polymers (e.g. ethylhexylglycerin, niacinamide, panthenol), thickeners (e.g. carbomer, xanthan gum), electrolytes (e.g. sodium chloride, sodium sulfate), emulsifiers (e.g. PEG-40 hydrogenated castor oil, butylene glycol palmitate), pH adjusting agents (e.g. triethanolamine, citric acid), perfuming agents (e.g. hydroxyisohexyl 3-cyclohexene carboxaldehyde, linalool, geraniol), sequestering agents (e.g. EDTA, tetrasodium EDTA), emollients (e.g. dimethicone, diisopropyl adipate), humectants (e.g. glycerin, lactic acid), lubricants (e.g. beeswax, paraffin), protein derivatives (e.g. hydrolyzed wheat protein, sericin), ethylene adducts (e.g. ethylene oxide), UV filters (e.g. ethylhexyl methoxycinnamate, benzophenone-4), volatile and non-volatile silicone fluids (e.g. cyclopentasiloxane, phenyl trimethicone), and isoparaffins (e.g. C₁₁₋₁₂ isoparaffin, C₁₈₋₇₀ isoparaffin).

In an embodiment of the invention said composition is suitable for application to a keratinous substrate. In certain embodiments said keratinous substrate is human hair.

In a second aspect of the invention there is provided the use of the composition of the first aspect of the invention as a hair fixative.

In a third aspect of the invention there is provided a method of making the personal care composition according to the first aspect of the invention comprising the steps of:
a. dissolving the natural film-forming biopolymer in a water-containing solvent to form a suspension;
b. adding the compound containing an amine moiety to the suspension;
c. adding the alcohol to the suspension to form the composition of the first aspect.

In a fourth aspect of the invention there is provided a method for styling hair comprising the steps of:
a. applying the composition of the first aspect of the invention to the hair; and
b. shaping the hair.

Throughout this specification wherever the term "by weight" is used with reference to the composition of the invention, this is to be understood as referring to the weight percentage as based on the total weight of the composition unless otherwise stated.

### BRIEF DESCRIPTION OF THE DRAWINGS

Embodiments of the invention are further described hereinafter with reference to the accompanying drawings, in which:
Figure 1 shows α-L-guluronic acid and β-D-mannuronic acid as found in alginic acid;
Figure 2 shows M blocks and G blocks in alginic acid;
Figure 3 shows α-D-galacturonic acid as found in pectin.

### DETAILED DESCRIPTION

The personal care composition of the invention comprises a natural film-forming biopolymer and a water-containing solvent. The natural film-forming biopolymer can be directly derived from plant or animal matter such that it can be provided for use in said personal care composition without need for covalent chemical modification.

In an embodiment, the composition of the invention comprises said biopolymer in an amount of about 0.1% to about 15% by weight of the composition. In another embodiment the composition of the invention comprises said biopolymer in an amount of about 0.1% to about 10% by weight of the composition and in other embodiments in an amount of about 0.1% to about 5% by weight of the composition.

The personal care composition of the invention can further comprise a compound containing an amine moiety. The inclusion of such a compound can ensure that films formed by the composition have a high clarity. This is in contrast to starch-based polymers used in personal care compositions of the prior art which produce opaque films. Opacity can often be a particularly undesirable property for personal care compositions such as hair styling products as consumers typically prefer products which exhibit clear films.

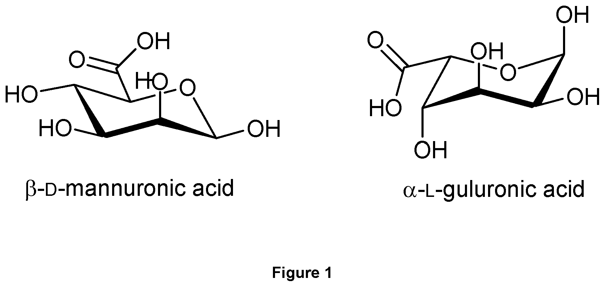

In some embodiments of the invention, the film-forming biopolymer of the invention is alginic acid. Alginic acid is a polysaccharide of (1-4)-linked β-D-mannuronate and α-L-guluronate (see Figure 1) extracted from 100% renewable sources such as brown seaweeds (Phaeophyta). Known applications of alginic acid include its use as a thickener in a variety of products including toothpastes and soaps. Furthermore, alginic acid can form stable gels in water allowing for use as a binding or emulsifying agent.

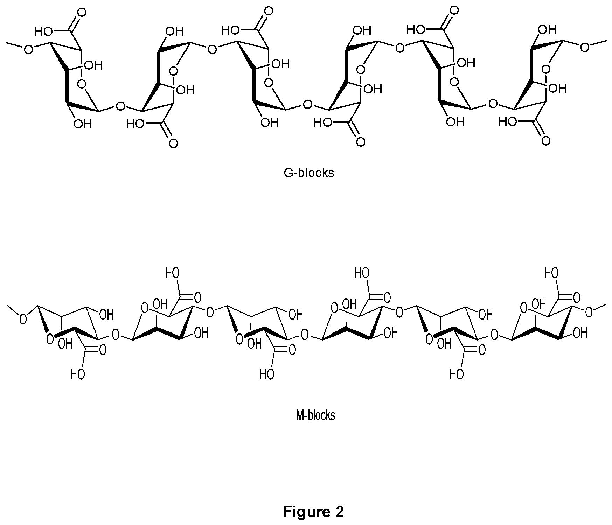

Examples of botanical sources of alginic acid are *Ascophyllum nodosum, Laminaria* spp., *Macrocystis pyrifera, Sargassum* spp., and *Undaria pinnatifida.* Investigations have shown (Haug, A et al. Carbohydr. Res. 1974, 32, 217 and Grasdalen, H. et al. Carbohydr. Res. 1981, 89, 179) that the polymer chain of alginic acid is made up of three kinds of regions or blocks, namely G blocks, M blocks and MG blocks. The G blocks contain only units derived from α-L-guluronic acid, the M blocks are based entirely on β-D-mannuronic acid and the MG blocks consist of alternating units of α-L-guluronic acid and β-D-mannuronic acid (see Figure 2). Because an M block is formed from equatorial groups at C-1 and C-4, it is a relatively straight polymer, like a flat ribbon. However, the G block is formed from axial groups at both C-1 and C-4 ensuring that the resulting chain is buckled. Each alginic acid molecule can thus be regarded as a block copolymer containing M, G, and MG blocks with the proportion of these blocks varying with the seaweed source.

Throughout this disclosure wherever the term "alginate" is used, this is to be understood as referring to alginic acid, of molecular weight typically between 5,000 to 800,000 g/mol and/or salts of said alginic acid. In some embodiments of the invention said alginic acid can thus be of molecular weight between 5,000 to 800,000 g/mol.

It has been shown that the physical properties of alginates depend on the relative proportion of the three types of blocks (Penman, A. and Sanderson, G.R. Carbohydr. Res. 1972, 25, 280). The alginate of greatest industrial importance is sodium alginate. Uses have also been found for the potassium, ammonium and calcium salts, as well as alginic acid itself. The only synthetic derivative of alginic acid to find wide use, and has found acceptance as a food additive, is propylene glycol alginate.

In other embodiments of the invention, the natural biopolymer is pectin.

Pectin is a natural polymer constituting the cell walls of most terrestrial plants, and it is extensively employed in the food industry, as a thickener or stabilizing agent. It is usually extracted from several types of fruits, with chemical or enzymatic methods and can have a molecular weight typically between 60 and 130,000 g/mol.

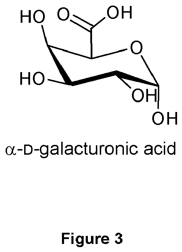

Pectin is composed of at least three polysaccharide domains: homogalacturonan (HGA), rhamnogalacturonan-I (RG-I) and rhamnogalacturonan-II (RG-II) (Yapo, B.M.; Carbohydrate Polym. 2011, 86, 373; Round, A.N.; Rigby, N.M.; MacDougall, A.J.; Morris, V.J. Carbohydrate Res. 2011, 345, 487; Mohnen, D. Current Opinion in Plant Biology, 2008, 11, 266). HGA is the major component of pectic polysaccharides, and contains (1-4)-linked α-D-galacturonate (see Figure 3) that are partially methyl-esterified and sometimes partially acetyl-esterified. The ratio of methyl-esterified residues of the HGA backbone to the total carboxylic acid units in the salt form is named degree of esterification (DE) (Monsoor, M.A.; Kalapathy, U.; Proctor, A. J. Agric. Food Chem. 2001, 49, 2756). Depending on the degree of esterification, pectins are classified as low methoxyl (LM, DE <50%) or high methoxyl (HM, DE >50%).

RG-I is composed of the disaccharide unit galacturonic acid-rhamnose, 20-80% of the rhamnose residues being substituted with neutral oligosaccharides, mainly arabinofuranose and galactose. Furthermore, fucose, glucopyranose and 4-O-methyl-glucopyranose may be found as terminal residues of the side chains. RG-II has a more complex structure: it presents a small backbone consisting of (1-4)-linked α-D-galacturonate residues, and side chains of different sugars such as rhamnose, galacturonic acid, galactose, arabinofuranose, fucose, apiofuranose and many others.

Pectin and alginic acid based compositions form films with different properties and solubilities.

Alginates can be made into two types of film which have different properties: water-soluble films (usually from sodium alginate) and oil-soluble films (usually from calcium alginate). Water-soluble films can be made by evaporation of a solution of alginate or by extrusion of an alginate solution into a non-solvent which mixes with water, such as acetone or ethanol. These films are impervious to grease, fats and waxes but allow water vapour to pass through. They are brittle when dry but can be plasticized with glycerol, sorbitol or urea.

Water-insoluble films can be made from alginates by treating a water-soluble film with a di- or trivalent cation (Ca²+ is most frequently used) or with acid. They can also be made by extrusion of a solution of a soluble alginate into a bath of a calcium salt. Some alginates, such as zinc alginate, are soluble in excess ammonia solution. If the ammonia is evaporated from a film of such a solution, an insoluble film of zinc alginate remains. These films of insoluble alginate are not water-repellent and will swell on prolonged exposure to water.

When powders of soluble alginates are wetted with water, the hydration of particles results in each having a tacky surface. Unless precautions are taken, the particles will rapidly stick together resulting in clumps which are very slow to completely hydrate and dissolve. The particle size and type will affect solubility behaviour.

The solubility of alginate in water is decreased if other compounds are present that may compete with the alginate for the water necessary for its hydration. The presence of sugars, starches or proteins in the water will reduce the rate of hydration and longer mixing times will be necessary. Similar effects are encountered by the presence of salts of monovalent cations at levels above about 0.5% by weight of the composition. All of these substances are best added after the alginate has been hydrated and dissolved. The presence of small quantities of many polyvalent cations inhibits the hydration of alginates and larger quantities cause precipitation.

Alginates are relatively insoluble in water-miscible solvents such as alcohols and ketones. Aqueous solutions (1%) of alginates typically tolerate the addition of 10-20% of these solvents and propylene glycol alginate tolerates 20-35% before precipitation. The presence of such solvents in water, before dissolving the alginate, will hinder hydration.

Pectins, like alginates, are relatively insoluble in water-miscible solvents such as alcohols and ketones. Aqueous solutions (1%) of pectins typically tolerate the addition of up to 10-20% of these solvents, before precipitation. As for alginates, the presence of water-miscible solvents such as alcohols or ketones in water before dissolving the pectin limits the extent of hydration.

Hence the presence of pectins and alginates in water-containing solvents results in problems associated with poor hydration of the biopolymer. The problem of poor hydration is further compounded by the inclusion of water-miscible solvents such as ethanol. The use of ethanol is highly desirable in hair styling compositions as its incorporation as part of the solvent system considerably decreases the drying time of such compositions following their application to a keratinous substrate when compared to water-only solvent systems. The poor hydration of pectin and alginate polymers in water-miscible solvents however effectively limits the quantity of ethanol to a maximum of 20% before precipitation occurs. The inclusion of the amine containing moiety in the composition of the present invention however ameliorates this problem. The biopolymer and the amine containing moiety interact to form an amine salt of the biopolymer in situ. Formation of the amine salt improves the hydration of the biopolymer. Consequently, compositions of the invention can tolerate much higher proportions of ethanol (greater than 20%) as part of the solvent system.

The compound containing an amine moiety of the composition of the invention may be selected from an alkylamine, a hydroxyalkylamine or an amino acid.

Thus in some embodiments, said compound containing an amine moiety is an amino acid or derivative thereof. The derivatives of said amino acid can include, but are not limited to, salts, zwitterions, polymers, esters or amides (including polyamides).

In an embodiment said compound containing an amine moiety is an alkylamine, a hydroxyalkylamine or a proteinogenic amino acid or derivative thereof.

In an embodiment, the compound containing an amine moiety may be an amine, diamine, triamine or a polyamine.

In an embodiment, the compound containing an amine moiety may be a primary amine, secondary amine or a tertiary amine.

In an embodiment, the compound containing an amine moiety may be a primary aminoalcohol, secondary aminoalcohol or tertiary aminoalcohol, wherein said primary aminoalcohol, secondary aminoalcohol or tertiary aminoalcohol have the general structure according to Formula (I), Formula (II) or Formula (III): 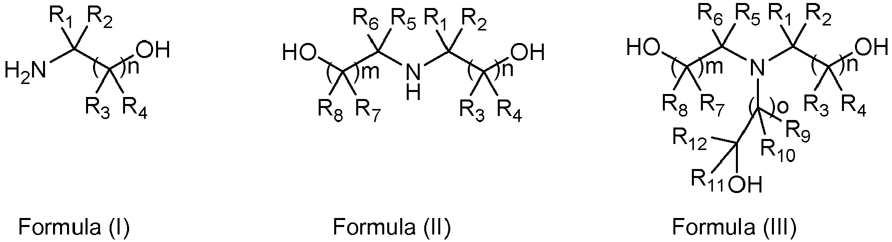 wherein R₁ to R₁₂ are each independently selected from the group consisting of: H, C₁₋₁₂ alkyl, aryl (e.g. phenyl), C₁₋₁₂ alkyl aryl and C₁₋₁₂ heteroalkyl wherein said C₁₋₁₂ heteroalkyl contains one of more heteroatoms selected from oxygen, nitrogen or sulphur; and m, n and o are each independently an integer from 0 to 14.

The above mentioned C₁₋₁₂ alkyl groups and any of the C₁₋₁₂ alkyl groups disclosed herein may be straight or branched chain alkyl groups and may have, for example, from 1 to 12, e.g. 6 carbon atoms. Thus C₁₋₁₂ means a moiety having 1, 2, 3, 4, 5, 6, 7, 8, 9, 10, 11 or 12 carbon atoms. The above mentioned C₁₋₁₂ heteroalkyl group comprises a C₁₋₁₂ alkyl group interrupted by one or more oxygen, sulphur or nitrogen atoms. The term "each independently selected from the group consisting of" in this context and as used herein is intended to mean that each of the listed 'R' groups may be selected from the group independently of the other 'R' groups (here the term 'R' group refers to any group R, R₁, R₂, R₃, R₄, R₅, R₆, R₇, R₈, R₉ etc.). Therefore, each 'R' group may be the same or different from each other.

In an embodiment, R₁ to R₁₂ of Formula (I), Formula (II) and Formula (III) can be selected from H.

In an embodiment, said compound containing an amine moiety is selected from the group consisting of: ethanolamine, ethanolamine derivatives, triethanolamine, triethanolamine derivatives, aminomethylpropanol, aminomethylpropanol derivatives, isopropanolamine, isopropanolamine derivatives, ethylene diamine, ethylene diamine derivatives, propylene diamine, propylene diamine derivatives, ethylenediaminetetraacetic acid (EDTA) and salts of ethylenediaminetetraacetic acid.

In an embodiment, said compound containing an amine moiety is triethanolamine or 2-amino-2-methyl-1-propanol.

In another embodiment, said compound containing an amine moiety is arginine, histidine or lysine and derivatives thereof. In a further embodiment said compound containing an amine moiety is proline or glycine and derivatives thereof. The derivatives of arginine, histidine, proline, glycine or lysine include, but are not limited to, salts, zwitterions, polymers, esters or amides (including polyamides).

In particular embodiments said compound containing an amine moiety is selected from sodium glycinate, sodium histidinate and sodium prolinate. Thus in some embodiments said compound containing an amine moiety can be an amino acid salt.

In some embodiments, said compound containing an amine moiety can be an amino acid and derivatives thereof, wherein the amino acid has the general structure according to Formula (IV): 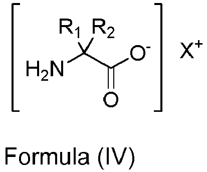 wherein R₁ and R₂ are each independently selected from the group consisting of: H, C₁₋₁₂ alkyl, aryl (e.g. phenyl), C₁₋₁₂ alkyl aryl and C₁₋₁₂ heteroalkyl wherein said C₁₋₁₂ heteroalkyl contains one or more heteroatoms selected from oxygen, nitrogen or sulphur, and wherein X⁺ is a counter ion. In an embodiment X⁺ can be hydrogen. The above mentioned C₁₋₁₂ alkyl groups may be optionally interrupted by one or more oxygen, sulphur or nitrogen atoms.

In an embodiment, the composition of the invention comprises said compound containing an amine moiety in an amount of about 0.1% to about 3% by weight of the composition.

The composition of the invention comprises an alcohol/water solvent mixture. The alcohol may be a monohydric alcohol with a boiling point below 100 °C such as ethanol. Water and ethanol mixtures form a positive azeotrope (95.6:4.4 ethanol:water), whereby the boiling temperature of the azeotrope is lower than the boiling temperature of either of its constituents. This azeotropic effect influences evaporation rates, meaning that an alcohol/water solvent mixture will evaporate more rapidly than water alone, hence drying time of the personal care compositions when applied to hair is significantly reduced in comparison with water-only solvent systems. Examples of the improvements in drying times observed for hair fixatives in alcohol/water solvent mixtures are provided in WO2005110341, wherein it was observed that drying time decreases (% weight loss with time increases) with increasing levels of ethanol, up to 35% by weight (the maximum limit of solubility for the polymer used).

Said alcohol/water solvent mixture comprises at least 15% alcohol by weight of the composition. In other embodiments, said mixture comprises at least 20% alcohol by weight of the composition. In further embodiments, said mixture may respectively comprise at least 30%, at least 40% or at least 50% alcohol by weight of the composition.

In an embodiment of the invention, said alcohol/water solvent mixture comprises 30% to 60% alcohol by weight of the composition. In other embodiments, said mixture comprises 40% to 60% alcohol by weight of the composition. In other embodiments, said mixture comprises about 45% to about 55% by weight of the composition and in further embodiments said mixture comprises about 50% to about 55% by weight of the composition. Thus in certain embodiments, the alcohol/water solvent mixture of the invention may comprise substantially higher quantities of alcohol than personal care compositions of the prior art which also contain natural biopolymers or naturally derived polymers.

The composition of the invention may also include a surfactant. In one embodiment the surfactant may be comprised in an amount of from 0.01% to 5%, in other embodiments from 0.01% to 1% and in further embodiments from 0.02% to 0.8% by weight of the composition. Surfactants are generally classified as non-ionic, anionic, cationic, amphoteric or zwitterionic according to their ionic behaviour in aqueous solution.

Examples of suitable non-ionic surfactants are condensation products of aliphatic (C8-C18) primary or secondary linear or branched chain alcohols or phenols with alkylene oxides, usually ethylene oxide and generally having from 6 to 30 ethylene oxide groups. Other suitable non-ionic surfactants include esters of sorbitol, esters of sorbitan anhydrides, esters of propylene glycol, fatty acid esters of polyethylene glycol, fatty acid esters of polypropylene glycol, ethoxylated esters and polyoxyethylene fatty ether phosphates.

Examples of suitable anionic surfactants are the alkyl sulfates, alkyl ether sulfates, alkaryl sulfonates, alkanoyl isethionates, alkyl succinates, alkyl sulfosuccinates, N-alkoyl sarcosinates, alkyl phosphates, alkyl ether phosphates, alkyl ether carboxylates, and alpha-olefin sulfonates, especially their sodium, magnesium ammonium and mono-, di- and triethanolamine salts. The alkyl and acyl groups generally contain from 8 to 18 carbon atoms and may be unsaturated. The alkyl ether sulfates, alkyl ether phosphates and alkyl ether carboxylates may contain from one to 10 ethylene oxide or propylene oxide units per molecule, and preferably contain 2 to 3 ethylene oxide units per molecule.

Examples of suitable cationic surfactants are cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethyibenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, (and the corresponding hydroxides thereof), and those materials having the CTFA designations Quaternium-5, Quaternium-31 and Quaternium-18.

Examples of suitable amphoteric and zwitterionic surfactants include alkyl amine oxides, alkyl betaines, alkyl amidopropyl betaines, alkyl sulfobetaines (sultaines), alkyl glycinates, alkyl carboxyglycinates, alkyl amphopropionates, alkylamphoglycinates alkyl amidopropyl hydroxysultaines, acyl taurates and acyl glutamates, wherein the alkyl and acyl groups have from 8 to 19 carbon atoms.

In an embodiment, the composition of the invention may comprise one or more non-ionic surfactants. In certain embodiments, non-ionic surfactants are selected from polyoxyethylene nonyl phenyl ether, Polysorbate 20, Polysorbate 80 and mixtures thereof. Further anionic, cationic, amphoteric and zwitterionic surfactants may suitably be used in conjunction with non-ionic surfactants in compositions of the present invention, to improve, for example, foaming power and/or foam stability. The one or more non-ionic surfactants may thus optionally be combined with one or more amphoteric surfactants. The amphoteric surfactants are preferably selected from lauryl amine oxide, cocodimethyl sulfopropyl betaine, lauryl betaine, sodium cocamphopropionate, and cocamidopropyl betaine. In an embodiment, the amphoteric surfactant is cocamidopropyl betaine.

The composition of the invention may comprise a non-hydrocarbon propellant suitable for aerosol delivery of the composition to the desired application surface. To provide the improved spray performance benefits, the composition should be substantially free of hydrocarbon propellants, e.g., contain less than about 5% by weight of the composition of such hydrocarbon propellants.

In one embodiment the composition of the present invention comprises less than about 5%, in other embodiments less than about 3% and in further embodiments zero percent of hydrocarbon propellants by weight of the composition. The spray performance of anhydrous aerosol hairspray compositions is improved by minimizing the concentration of the hydrocarbon propellants to less than about 5% by weight of the composition. The term "hydrocarbon propellants" as used herein refers to those liquefiable gases that contain only carbon and hydrogen atoms, most notably of which are propane, butane, and isobutane.

The total concentration of the non-hydrocarbon propellant in the composition of the present invention may comprise one or more non-hydrocarbon propellants. In one embodiment, the total non-hydrocarbon propellant concentration may be from about 5% to about 50%, in other embodiments from about 15% to about 40% and in further embodiments from about 25% to about 40%, by weight of the composition. The term "non-hydrocarbon propellant" as used herein refers to all liquefiable gases suitable for use in topical application to human hair or skin, excluding the above-identified hydrocarbon propellants. Examples of suitable non-hydrocarbon propellants include, but are not limited to, nitrogen, carbon dioxide, nitrous oxide, atmospheric gas, 1,2-difluoroethane (Hydrofluorocarbon 152A) supplied as Dymel 152A by DuPont, dimethylether, and mixtures thereof. In an embodiment, the non-hydrocarbon propellant is dimethylether.

The composition of the present invention may further comprise optional components known or otherwise effective for use in hair care or personal care products with the proviso that the optional components are physically and chemically compatible with said composition and do not otherwise unduly impair the stability, aesthetics or performance. In one embodiment the optional components are comprised in an amount of from zero to about 25%, in other embodiments from about 0.05% to about 25% and in further embodiments from about 0.1% to about 15%, by weight of the composition.

Examples of optional components include, but are not limited to, preservatives (e.g. sodium benzoate, phenoxyethanol, methylisothiazolinone), surfactants, conditioning or styling polymers (e.g. ethylhexylglycerin, niacinamide, panthenol) other than and in addition to the natural biolpolymers described herein, thickeners and viscosity modifiers (e.g. carbomer, xanthan gum), electrolytes (e.g. sodium chloride, sodium sulfate), fatty alcohols, emulsifiers (e.g. PEG-40 hydrogenated castor oil, butylene glycol palmitate), pH adjusting agents (e.g. triethanolamine, citric acid), perfume oils, perfume solubilising agents, perfuming agents (e.g. hydroxyisohexyl 3-cyclohexene carboxaldehyde, linalool, geraniol), sequestering agents (e.g. EDTA, tetrasodium EDTA), emollients (e.g. dimethicone, diisopropyl adipate), lubricants and penetrants such as various lanolin compounds, protein hydrolysates and other protein derivatives (e.g. hydrolyzed wheat protein, sericin), ethylene adducts (e.g. ethylene oxide) and polyoxyethylene cholesterol, sunscreens and UV filters (e.g. ethylhexyl methoxycinnamate, benzophenone-4), volatile and non-volatile silicone fluids (e.g. cyclopentasiloxane, phenyl trimethicone), and isoparaffins (e.g. C₁₁₋₁₂ isoparaffin, C₁₈₋₇₀ isoparaffin).

The composition of the invention may suitably be in a gel, cream, a pump spray or aerosol form. A particularly preferred product form is a pump spray. In embodiments wherein the composition is a cream or a gel, the composition may further comprise a structurant or thickener. In one embodiment the structurant or thickener may be comprised in an amount of from 0.1% to 10% and in other embodiments from 0.5% to 3% by weight of the composition. Examples of suitable structurants or thickeners are polymeric thickeners such as carboxyvinyl polymers. A carboxyvinyl polymer is an interpolymer of a monomeric mixture comprising a monomeric olefinically unsaturated carboxylic acid, and from about 0.01% to about 10% by weight of the total monomers of a polyether of a polyhydric alcohol. Natural structurants are particularly preferred, such as water soluble or colloidally water soluble polymers like cellulose ethers (e.g. methylcellulose, hydroxyethylcellulose, hydroxypropylmethylcellulose and carboxymethylcellulose), guar gum, sodium alginate, gum arabic, xanthan gum, polyvinyl alcohol, polyvinyl pyrrolidone, hydroxypropyl guar gum, starch and starch derivatives, and other thickeners, viscosity modifiers, gelling agents, etc. It is also possible to use inorganic thickeners such as bentonite or laponite clays.

The composition of the present invention may also comprise an aerosol hair mousse, which is emitted from the aerosol container as a foam which is then typically worked through the hair with fingers or a hair styling tool and either left on the hair or rinsed out.

In an embodiment, the composition of the invention is prepared by first dissolving the natural biopolymer in a water-containing solvent and then adding the compound containing an amine moiety or the compound containing a quaternary ammonium moiety to the suspension. In embodiments of the invention wherein the solvent of the composition is an alcohol/water mixture, alcohol is added to the suspension after the addition of the compound containing the amine moiety or the compound containing a quaternary ammonium moiety. Any optional additional ingredients may also be added after the compound containing the amine moiety or the compound containing a quaternary ammonium moiety has been added. Thus in embodiments wherein the solvent is an alcohol/water mixture, said optional ingredients can be added either before or after the addition of the alcohol.

Following its preparation, the composition of the invention can then be packaged into a suitable container such as a pump-spray or an aerosol dispenser. The composition can be contained or dispensed in any known or otherwise effective container or delivery system. All such containers or delivery systems should be compatible with the essential and any selected optional ingredients of the composition.

### EXAMPLES

Exemplary compositions of the personal care compositions of the present invention are shown in Table 1, Table 2, Table 3 and Table 4 and were formulated in accordance with the general methods detailed below.

For examples 1 to 4, alginic acid was added to water with stirring until a fine colloidal suspension was obtained. To this suspension either 2-amino-2-methyl-1-propanol (see examples 1 and 2) or triethanolamine (see examples 3 and 4) was added with stirring, at which point the amine alginate polymer was formed that dissolved fully in the water, providing a solution with high clarity. Ethanol was slowly added to this aqueous solution of amine alginate with stirring, until the requisite amount of ethanol had been added. The resultant composition was ready for application to hair. On application to hair, the samples dried quickly and had stiff hold. The polymers were easily removed from the hair with shampoo.

For example 5, the amine alginate polymer was formed as described for examples 3 to 4, but no ethanol was added. The resultant composition was ready for application to hair. On application to hair, the sample dried more slowly than that for examples 1 to 4, but still provided stiff hold. The polymer was easily removed from the hair with shampoo.

For example 6, pectin was added to water with stirring until a fine colloidal suspension was obtained. To this suspension triethanolamine was added, at which point the amine pectinate polymer was formed that dissolved fully in the water, providing a solution with high clarity. Ethanol was slowly added to this aqueous solution of amine pectinate with stirring, until the requisite amount of ethanol had been added. The resultant composition was a thick gel and ready for application to hair. On application to hair, the sample dried quickly and had stiff hold. The polymer was easily removed from the hair with shampoo.

For examples 7 to 10, alginic acid was added to water with stirring until a fine colloidal suspension was obtained. To this suspension either choline hydroxide aqueous solution (see comparative example 7) or sodium glycinate aqueous solution (see example 8) or sodium histidinate aqueous solution (see example 9) or sodium prolinate aqueous solution (see example 10) was added with stirring, at which point the amine alginate polymer was formed that dissolved fully in the water, providing a solution with high clarity. Ethanol was slowly added to this aqueous solution of amine alginate with stirring, until the requisite amount of ethanol had been added. The resultant composition was ready for application to hair. On application to hair, the samples dried quickly and had stiff hold. The polymers were easily removed from the hair with shampoo.

**Table 1**

| **EXAMPLES** | | **EXAMPLE 1** | **EXAMPLE 2** | **EXAMPLE 3** |
|---|---|---|---|---|
| **Formulation Ingredients** | | Alginic acid 2.0% w/w | Alginic acid 4.0% w/w | Alginic acid 2.05% w/w |
| | | 2-amino-2-methyl-1-propanol 0.85% w/w | 2-amino-2-methyl-1-propanol 1.7% w/w | Triethanolamine 1.45% w/w |
| | | Ethanol 53.4% w/w | Ethanol 52.3% w/w | Ethanol 53.0% w/w |
| | | Water 43.7% w/w | Water 42.7% w/w | Water 43.5% w/w |
| **Stiffness** | **1 = soft >>> 5 = stiff** | 5 | 5 | 5 |
| **Flaking** | **1 = most >>> 5 = none** | 3.5 | 2 | 4.5 |
| **Flyaway / Static** | **1 = full fan (worst) >>>** | 4.5 | 4.5 | 5 |
| | **5 = no fan(best)** | | | |
| **Feel** | **1= coated >>> 5 = natural** | 4 | 4 | 4 |
| **Shine** | **1= dull >>>** | 4.5 | 4 | 4.5 |
| | **5 = Very shiny** | | | |
| **Tack** | **1= High >>> 5** = **low** | 5 | 5 | 4 |
| **Sprayability/ Spreadability** | **Visual, good or bad** | good, even spray | good spray, strong centre stream | good spray |
| **Film** | **Visual, good or bad** | excellent | excellent | slightly hazy |
| **Curl Memory (After 20 extensions)** | **Initial curled length (cm)** | 8.9 | 7.6 | 7.6 |
| | **Curled length After (cm)** | 11.4 | 14.0 | 10.2 |
| | **% curl retention (based on straight length of 33cm)** | 89.5% | 75.0% | 90.0% |
| **Curl Definition** | **visual, good or bad** | good | good | good |
| **Curl Retention (90% RH/32°C)** | **Initial Length (cm)** | 7.6 | 5.1 | 6.4 |
| | **Length after 10 min (cm)** | 14.0 | 7.6 | 10.2 |
| | **% curl retention (based on straight length of 33cm)** | 75.0% | 90.9% | 85.7% |
| | **Length after 60 min (cm)** | 17.8 | 16.5 | 19.1 |
| | **% curl retention (based on straight length of 33cm)** | 60.0% | 59.1% | 52.4% |

**Table 2**

| **EXAMPLES** | | **EXAMPLE 4** | **EXAMPLE 5** | **EXAMPLE 6** |
|---|---|---|---|---|
| **Formulation Ingredients** | | Alginic acid 4.1% w/w | Alginic acid 4.1% w/w | Pectin 2.05% w/w |
| | | Triethanolamine 2.9% w/w | Triethanolamine 2.9% w/w | Triethanolamine 1.45% w/w |
| | | Ethanol 51.1% w/w | --- | Ethanol 53.0% w/w |
| | | Water 41.8% w/w | Water 93.0% w/w | Water 43.5% w/w |
| **Stiffness** | **1 = soft >>> 5 = stiff** | 5 | 5 | 5 |
| **Flaking** | **1 = most >>> 5 = none** | 2 | 2 | 4 |
| **Flyaway / Static** | **1 = full fan (worst) >>>** | 4.5 | 4.5 | 4.5 |
| | **5 = no fan(best)** | | | |
| **Feel** | **1= coated >>> 5 = natural** | 4 | 3 | 3 |
| **Shine** | **1= dull >>>** | 3.5 | 3.5 | 4 |
| | **5** = **Very shiny** | | | |
| **Tack** | **1= High >>> 5 = low** | 5 | 5 | 5 |
| **Sprayability/ Spreadability** | **Visual, good or bad** | good, strong centre stream | good, strong centre stream | not sprayable, good spreadability |
| **Film** | **Visual, good or bad** | excellent | excellent | excellent |
| **Curl Memory (After 20 extensions)** | **Initial curled length (cm)** | 5.1 | 6.7 | 7.5 |
| | **Curled length After (cm)** | 11.4 | 13.2 | 11.0 |
| | **% curl retention (based on straight length of 33cm)** | 77.3% | 75.3% | 86.3% |
| **Curl Definition** | **visual, good or bad** | good | good | good |
| **Curl Retention RH/32°C)** | **Initial Length (cm)** | 5.1 | 6.7 | 7.5 |
| | **Length after 10 min (cm)** | 12.7 | 14.1 | 12.9 |
| | **% curl retention (based on straight length of 33cm)** | 72.8% | 71.8% | 78.8% |
| | **Length after 60 min (cm)** | 22.9 | 21.0 | 22.3 |
| | **% curl retention (based on straight length of 33cm)** | 36.4% | 45.6% | 42.0% |

**Table 3**

| **EXAMPLES** | | **COMPARATIVE EXAMPLE 7** | **EXAMPLE 8** | **EXAMPLE 9** |
|---|---|---|---|---|
| **Formulation Ingredients** | | Alginic acid 2.00% w/w | Alginic acid 2.00% w/w | Alginic acid 2.00% w/w |
| | | Choline hydroxide 2.65% w/w | Sodium glycinate 1.00% w/w | Sodium histidinate 1.83% w/w |
| | | Ethanol 47.67% w/w | Ethanol 48.50% w/w | Ethanol 48.09% w/w |
| | | Water 47.67% w/w | Water 48.50% w/w | Water 48.09% w/w |
| **Stiffness** | **1 = soft >>> 5 = stiff** | 5 | 5 | 5 |
| **Flaking** | **1 = most >>> 5 = none** | 4 | 3 | 3 |
| **Flyaway / Static** | **1 = full fan (worst) >>>** | 5 | 5 | 5 |
| | **5 = no fan(best)** | | | |
| **Feel** | **1= coated >>> 5 = natural** | 4 | 4.5 | 4.5 |
| **Shine** | **1= dull >>>** | 5 | 3 | 3 |
| | **5 = Very shiny** | | | |
| **Tack** | **1= High >>> 5 = low** | 4 | 4 | 4 |
| **Sprayability/ Spreadability** | **Visual, good or bad** | Good spray | Good spray | Good spray |
| **Film** | **Visual, good or bad** | excellent | good | good |
| **Curl Memory (After 20 extensions)** | **Initial curled length (cm)** | ND | ND | ND |
| | **Curled length After (cm)** | ND | ND | ND |
| | **% curl retention (based on straight length of 33cm)** | ND | ND | ND |
| **Curl Definition** | **visual, good or bad** | good | good | good |
| **Curl Retention (90% RH/32°C)** | **Initial Length (cm)** | ND | ND | ND |
| | **Length after 10 min (cm)** | ND | ND | ND |
| | **% curl retention (based on straight length of 33cm)** | ND | ND | ND |
| | **Length after 60 min (cm)** | ND | ND | ND |
| | **% curl retention (based on straight length of 33cm)** | ND | ND | ND |

| | | | | |
|---|---|---|---|---|
| ND: not determined | | | | |

**Table 4**

| **EXAMPLES** | | **EXAMPLE 10** |
|---|---|---|
| **Formulation Ingredients** | | Alginic acid 2.00% w/w |
| | | Sodium prolinate 1.41% w/w |
| | | Ethanol 48.29% w/w |
| | | Water 48.29% w/w |
| **Stiffness** | **1 = soft >>> 5 = stiff** | 5 |
| **Flaking** | **1 = most >>> 5 = none** | 4 |
| **Flyaway / Static** | **1 = full fan (worst) >>>** | 5 |
| | **5 = no fan(best)** | |
| **Feel** | **1= coated >>> 5 = natural** | 4 |
| **Shine** | **1= dull >>>** | 5 |
| | **5 = Very shiny** | |
| **Tack** | **1= High >>> 5 = low** | 4 |
| **Sprayability/ Spreadability** | **Visual, good or bad** | Good spray |
| **Film** | **Visual, good or bad** | good |
| **Curl Memory (After 20 extensions)** | **Initial curled length (cm)** | ND |
| | **Curled length After (cm)** | ND |
| | **% curl retention (based on straight length of 33cm)** | ND |
| **Curl Definition** | **visual, good or bad** | good |
| **Curl Retention (90% RH/32°C)** | **Initial Length (cm)** | ND |
| | **Length after 10 min (cm)** | ND |
| | **% curl retention (based on straight length of 33cm)** | ND |
| | **Length after 60 min (cm)** | ND |
| | **% curl retention (based on straight length of 33cm)** | ND |

| | | |
|---|---|---|
| ND: not determined | | |

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

The reader's attention is directed to all papers and documents which are filed concurrently with or previous to this specification in connection with this application and which are open to public inspection with this specification.

## Claims

1. A personal care composition comprising a natural film-forming biopolymer and a water-containing solvent wherein said composition further comprises a compound containing an amine moiety and wherein said solvent is an alcohol/water mixture wherein said mixture comprises at least 15% alcohol by weight of the composition; wherein said alcohol has a boiling point of less than 100 °C; and wherein said biopolymer is alginic acid or pectin.

2. The composition of claim 1 wherein said biopolymer can react with said compound containing an amine moiety to form a salt.

3. The composition claim 1 or claim 2 wherein said compound containing an amine moiety is an alkylamine, a hydroxyalkylamine or an amino acid or wherein said compound containing an amine moiety is selected from the group consisting of: an amine, diamine, triamine, polyamine, aminoalcohol, diaminoalcohol, triaminoalcohol and polyaminoalcohol.

4. The composition of any preceding claim wherein said compound containing an amine moiety is selected from the group consisting of: ethanolamine, ethanolamine derivatives, triethanolamine, triethanolamine derivatives, aminomethylpropanol, aminomethylpropanol derivatives, isopropanolamine, isopropanolamine derivatives, ethylene diamine, ethylene diamine derivatives, propylene diamine, propylene diamine derivatives, ethylenediaminetetraacetic acid and salts of ethylenediaminetetraacetic acid or wherein said compound containing an amine moiety is triethanolamine or 2-amino-2-methyl-1-propanol or wherein said compound containing an amine moiety is arginine, histidine, proline, glycine or lysine and derivatives thereof wherein said derivatives of arginine, histidine, proline, glycine or lysine include salts, zwitterions, polymers, esters or amides.

5. The composition of any preceding claim comprising said biopolymer in an amount of about 0.1% to 15% by weight of the composition.

6. The composition of any preceding claim comprising said compound containing an amine moiety in an amount of about 0.1% to about 3% by weight of the composition.

7. The composition of claim 1 wherein said mixture comprises at least 20% alcohol by weight of the composition.

8. The composition of claim 1 wherein said mixture comprises 30% to 60% alcohol by weight of the composition and optionally wherein said mixture comprises about 45% to about 55% alcohol by weight of the composition.

9. The composition of any preceding claim wherein said alcohol is ethanol.

10. The composition according to any preceding claim comprising at least 20% water by weight of the composition.

11. The composition of any preceding claim suitable for application to a keratinous substrate wherein the keratinous substrate is human hair and optionally wherein said composition is substantially free of synthetic polymers or petrochemically derived components.

12. The use of the composition of any preceding claim as a hair fixative.

13. A method of making the composition of claim 1 comprising the steps of:
a. dissolving the natural film-forming biopolymer in a water-containing solvent to form a suspension;
b. adding the compound containing an amine moiety to the suspension; and further comprising the step:
c. adding the alcohol to the suspension to form the composition of claim 1.

14. A method of styling hair comprising the steps of:
a. applying the composition of any of claims 1 to 11 to the hair; and
b. shaping the hair.

## Patentansprüche

1. Körperpflegezusammensetzung, die ein natürliches filmbildendes Biopolymer und ein Wasser enthaltendes Lösungsmittel umfasst, wobei die Zusammensetzung ferner eine Verbindung umfasst, die einen Aminrest enthält, und wobei es sich bei dem Lösungsmittel um ein Gemisch aus Alkohol und Wasser handelt, wobei das Gemisch mindestens 15 Gew.-% Alkohol, bezogen auf das Gewicht der Zusammensetzung, umfasst und wobei der Alkohol einen Siedepunkt von weniger als 100 °C hat; und wobei es sich bei dem Biopolymer um Alginsäure oder Pektin handelt.

2. Zusammensetzung nach Anspruch 1, wobei das Biopolymer mit der einen Aminrest enthaltenden Verbindung unter Bildung eines Salzes umgesetzt werden kann.

3. Zusammensetzung nach Anspruch 1 oder Anspruch 2, wobei es sich bei der einen Aminrest enthaltenden Verbindung um eine Alkylamin-, eine Hydroxyalkylamin- oder eine Aminosäure handelt, oder wobei die einen Aminrest enthaltende Verbindung ausgewählt ist aus der Gruppe bestehend aus: einem Amin, Diamin, Triamin, Polyamin, Aminoalkohol, Diaminoalkohol, Triaminoalkohol und Polyaminoalkohol.

4. Zusammensetzung nach einem der vorhergehenden Ansprüche, wobei die einen Aminrest enthaltende Verbindung ausgewählt ist aus der Gruppe bestehend aus: Ethanolamin, Ethanolaminderivaten, Triethanolamin, Triethanolaminderivaten, Aminomethylpropanol, Aminomethylpropanolderivaten, Isopropanolamin, Isopropanolaminderivaten, Ethylendiamin, Ethylendiaminderivaten, Propylendiamin, Propylendiaminderivaten, Ethylendiamintetraessigsäure und Salzen von Ethylendiamintetraessigsäure, oder wobei es sich bei der einen Aminrest enthaltenden Verbindung um Triethanolamin oder 2-Amino-2-methyl-1-propanol handelt, oder wobei es sich bei der einen Aminrest enthaltenden Verbindung um Arginin, Histidin, Prolin, Glycin oder Lysin und Derivate davon handelt, wobei die Derivate von Arginin, Histidin, Prolin, Glycin oder Lysin Salze, Zwitterionen, Polymere, Ester oder Amide beinhalten.

5. Zusammensetzung nach einem der vorhergehenden Ansprüche, die das Biopolymer in einer Menge von etwa 0,1 Gew.-% bis 15 Gew.-%, bezogen auf das Gewicht der Zusammensetzung, umfasst.

6. Zusammensetzung nach einem der vorhergehenden Ansprüche, die die einen Aminrest enthaltende Verbindung in einer Menge von etwa 0,1 Gew.-% bis etwa 3 Gew.-%, bezogen auf das Gewicht der Zusammensetzung, umfasst.

7. Zusammensetzung nach Anspruch 1, wobei das Gemisch mindestens 20 Gew.-% Alkohol, bezogen auf das Gewicht der Zusammensetzung, umfasst.

8. Zusammensetzung nach Anspruch 1, wobei das Gemisch 30 Gew.-% bis 60 Gew.-% Alkohol, bezogen auf das Gewicht der Zusammensetzung, umfasst, und wobei das Gemisch gegebenenfalls etwa 45 Gew.-% bis etwa 55 Gew.-% Alkohol, bezogen auf das Gewicht der Zusammensetzung, umfasst.

9. Zusammensetzung nach einem der vorhergehenden Ansprüche, wobei es sich bei dem Alkohol um Ethanol handelt.

10. Zusammensetzung nach einem der vorhergehenden Ansprüche, die mindestens 20 Gew.-% Wasser, bezogen auf das Gewicht der Zusammensetzung, umfasst.

11. Zusammensetzung nach einem der vorhergehenden Ansprüche, die zur Anwendung auf einem keratinhaltigen Substrat geeignet ist, wobei es sich bei dem keratinhaltigen Substrat um menschliches Haar handelt, und wobei die Zusammensetzung gegebenenfalls frei von synthetischen Polymeren oder petrochemisch abgeleiteten Bestandteilen ist.

12. Verwendung der Zusammensetzung nach einem der vorhergehenden Ansprüche als Haarfixiermittel.

13. Verfahren zum Herstellen der Zusammensetzung nach Anspruch 1, das die folgenden Schritte umfasst:
a. Lösen des natürlichen filmbildenden Biopolymers in einem Wasser enthaltenden Lösungsmittel
zum Bilden einer Suspension;
b. Zugeben der einen Aminrest enthaltenden Verbindung zu der Suspension;
und das ferner den folgenden Schritt umfasst:
c. Zugeben des Alkohols zu der Suspension zum Bilden der Zusammensetzung nach Anspruch 1.

14. Verfahren zum Frisieren von Haar, das die folgenden Schritte umfasst:
a. Aufbringen der Zusammensetzung nach einem der Ansprüche 1 bis 11 auf das Haar; und
b. Formen des Haars.

## Revendications

1. Composition d'hygiène personnelle comprenant un biopolymère de formation de film naturel et un solvant contenant de l'eau dans laquelle ladite composition comprend en outre un composé contenant un groupement amine et dans laquelle ledit solvant est un mélange alcool/eau dans laquelle ledit mélange comprend au moins 15 % d'alcool en poids de la composition ; dans laquelle ledit alcool a un point d'ébullition de moins de 100 °C ; et dans laquelle ledit biopolymère est l'acide alginique ou la pectine.

2. Composition selon la revendication 1 dans laquelle ledit biopolymère peut réagir avec ledit composé contenant un groupement amine pour former un sel.

3. Composition selon la revendication 1 ou la revendication 2 dans laquelle ledit composé contenant un groupement amine est une alkylamine, une hydroxyalkylamine ou un acide aminé ou dans laquelle ledit composé contenant un groupement amine est sélectionné dans le groupe constitué par: une amine, une diamine, une triamine, une polyamine, un aminoalcool, un diaminoalcool, un triaminoalcool et un polyaminoalcool.

4. Composition selon l'une quelconque des revendications précédentes dans laquelle ledit composé contenant un groupement amine est sélectionné parmi le groupe constitué par :
l'éthanolamine, des dérivés de l'éthanolamine, la triéthanolamine, des dérivés de la triéthanolamine, l'aminométhylpropanol, des dérivés de l'aminométhylpropanol, l'isopropanolamine, des dérivés de l'isopropanolamine, l'éthylènediamine, des dérivés de l'éthylènediamine, la propylènediamine, des dérivés de la propylènediamine, l'acide éthylènediaminetétraacétique et des sels d'acide éthylènediaminetétraacétique ou dans laquelle ledit composé contenant un groupement amine est la triéthanolamine ou le 2-amino-2-méthyl-1-propanol ou dans laquelle ledit composé contenant un groupement amine est l'arginine, l'histidine, la proline, la glycine ou la lysine et des dérivés de celles-ci dans laquelle lesdits dérivés de l'arginine, l'histidine, la proline, la glycine ou la lysine incluent des sels, des zwitterions, des polymères, des esters ou des amides.

5. Composition selon l'une quelconque des revendications précédentes comprenant ledit biopolymère dans une quantité d'environ 0,1 % à 15 % en poids de la composition.

6. Composition selon l'une quelconque des revendications précédentes comprenant ledit composé contenant un groupement amine dans une quantité d'environ 0,1 % à environ 3 % en poids de la composition.

7. Composition selon la revendication 1 dans laquelle ledit mélange comprend au moins 20 % d'alcool en poids de la composition.

8. Composition selon la revendication 1 dans laquelle ledit mélange comprend de 30 % à 60 % d'alcool en poids de la composition et facultativement dans laquelle ledit mélange comprend d'environ 45 % à environ 55 % d'alcool en poids de la composition.

9. Composition selon l'une quelconque des revendications précédentes dans laquelle ledit alcool est l'éthanol.

10. Composition selon l'une quelconque des revendications précédentes comprenant au moins 20 % d'eau en poids de la composition.

11. Composition selon l'une quelconque des revendications précédentes adaptée à une application sur un substrat kératineux dans laquelle le substrat kératineux est un cheveux humain et facultativement ladite composition est substantiellement exempte de polymères synthétiques ou de composants dérivés de la pétrochimie.

12. Utilisation de la composition selon l'une quelconque des revendications précédentes comme un fixateur pour cheveux.

13. Procédé de fabrication de la composition selon la revendication 1 comprenant les étapes consistant à :
a. dissoudre le biopolymère de formation de film naturel dans un solvant contenant de l'eau pour former une suspension ;
b. ajouter le composé contenant un groupement amine à la suspension ;
et comprenant en outre l'étape consistant à :
c. ajouter l'alcool à la suspension pour former la composition selon la revendication 1.

14. Procédé de coiffure des cheveux comprenant les étapes consistant à :
a. appliquer la composition selon l'une quelconque des revendications 1 à 11 sur les cheveux ; et
b. mettre en forme les cheveux.
